# EUROPEAN PATENT SPECIFICATION

(11) **EP 2 043 559 B1**
(45) Date of publication and mention of the grant of the patent: **13.11.2019**
(21) Application number: 07812502.8
(22) Date of filing: 29.06.2007
(51) Int. Cl.: A61F 2/24

(54) **SYSTEM FOR DEPLOYING BALLOON-EXPANDABLE HEART VALVE**
SYSTEM FÜR DEN EINSATZ EINER BALLONEXPANDIERBAREN HERZKLAPPE
SYSTÈME POUR LE DÉPLOIEMENT D'UNE VALVE CARDIAQUE DILATABLE PAR UN BALLONNET

(30) Priority: 18.07.2006 US 488510
(43) Date of publication of application: 08.04.2009
(73) Proprietor: Edwards Lifesciences Corporation, Irvine, CA 92614 (US)
(72) Inventor: PATZ, Tim, Newport Beach, CA 92663 (US); PATTERSON, Marlowe, Orange, CA 92867 (US); KHANNA, Rajesh, Tustin, CA 92780 (US); LAU, Jackie, Anaheim, CA 92808 (US); ZHU, Mike, Irvine, CA 92604 (US); HSINGCHING, Crystal, Hsu, Newport Beach, CA 92663 (US)
(74) Representative: Eisenführ Speiser
(86) International application number: PCT/US2007/072549
(87) International publication number: WO 2008/011261

(56) References cited:
- EP-A- 1 254 644
- WO-A-2004/058097
- US-A1- 2004 210 304
- US-A1- 2006 149 360
- US-B2- 6 730 118

## Description

### Field of the Invention

The present invention relates to systems for implanting expandable prosthetic heart valves and, in particular, a shaped expansion member for deploying such heart valves.

### Background of the Invention

Heart valve replacement may be indicated when there is a narrowing of the native heart valve, commonly referred to as stenosis, or when the native valve leaks or regurgitates, such as when the leaflets are calcified. In one therapeutic solution, the native valve may be excised and replaced with either a biologic or a mechanical valve. Prosthetic valves attach to the patient's fibrous heart valve annulus, with or without the leaflets being present.

Conventional heart valve surgery is an open-heart procedure that is highly invasive, resulting in significant risks include bleeding, infection, stroke, heart attack, arrhythmia, renal failure, adverse reactions to the anesthesia medications, as well as sudden death. Fully 2-5% of patients die during surgery. The average hospital stay is between 1 to 2 weeks, with several more weeks to months required for complete recovery.

In recent years, advancements in "minimally-invasive" surgery and interventional cardiology have encouraged some investigators to pursue replacement of heart valves using remotely-implanted expandable valves without opening the chest or putting the patient on cardiopulmonary bypass. For instance, Percutaneous Valve Technologies ("PVT") of Fort Lee, N.J. and Edwards Lifesciences of Irvine, CA, have developed a balloon-expandable stent integrated with a bioprosthetic valve having flexible leaflets. The stent/valve device, marketed under the name Cribier-Edwards™ Aortic Percutaneous Heart Valve, is deployed across the native diseased valve to permanently hold the valve open, thereby alleviating a need to excise the native valve. The device is designed for percutaneous delivery in a cardiac catheterization laboratory under local anesthesia using fluoroscopic guidance, thereby avoiding general anesthesia and open-heart surgery. Other percutaneously- or surgically-delivered expandable valves are also being tested. For the purpose of inclusivity, the entire field will be denoted herein as the delivery and implantation of expandable valves.

Expandable heart valves use either balloon-or self-expanding stents as anchors. The uniformity of contact between the expandable valve and surrounding annulus, with or without leaflets, should be such that no paravalvular leakage occurs, and therefore proper expansion is very important. Perhaps more problematic is the quality of coaptation of the leaflets once the valve is expanded into place. Coaptation refers to the degree that the individual flexible leaflets come together in the valve orifice to occlude flow. If the leaflets do not quite meet, which could happen if the flexible valve is not expanded properly, regurgitation may occur. These and other issues make proper implant of the valve extremely critical. However, unlike open-heart surgery, the implant site is not directly accessible and the valve must be implanted remotely on the end of a catheter or cannula under indirect visualization (e.g., fluoroscopic imaging). It goes without saying that any system that improves the percentage of successful implants is desirable.

Balloon-expandable heart valves typically require expansion with a cylindrical balloon of clear nylon. The inflation fluid consists of saline mixed with a more viscous contrast media. The inherent viscosity of the mixture increases the inflation/deflation time, which is undesirable because the balloon occludes the target annulus when in use, and more and more procedures are being done off-pump, or on a beating heart.

Due to the intense current interest in expandable prosthetic heart valves, there is a need for implantation systems and techniques that reduce the time for and increase the chances of a successful implant.

US patent application 2004/0210304 shows a prosthetic valve assembly for use in replacing a deficient native valve, comprising a replacement valve supported on an expandable valve support. If desired, one or more anchors may be used. The valve support, which entirely supports the valve annulus, valve leaflets, and valve commissure points, is configured to be collapsible for transluminal delivery and expandable to contact the anatomical annulus of the native valve when the assembly is properly positioned. The anchor engages the lumen wall when expanded and prevents substantial migration of the valve assembly when positioned in place. The prosthetic valve assembly is compressible about a catheter, and restrained from expanding by an outer sheath. The catheter may be inserted inside a lumen within the body, such as the femoral artery, and delivered to a desired location, such as the heart. When the outer sheath is retracted, the prosthetic valve assembly expands to an expanded position such that the valve and valve support expand at the implantation site and the anchor engages the lumen wall. Active expansion may be provided by an expansion device such as a balloon.

### Summary of the Invention

The present invention provides a system for deploying balloon-expandable (i.e., plastically-expandable) prosthetic heart valves so that they assumed their desired operational shape. The system includes an expansion member that accommodates non-uniform expansion resistance in the heart valve to expanded to its desired tubular or other shape. The heart valve may have substantially more structural elements adjacent one end, typically the inflow end, and the expansion member may be tapered so as to expand the inflow end before the outflow so that the valve ends up in a tubular shape.

One aspect of the invention is a prosthetic heart valve implantation system comprising a balloon-expandable prosthetic heart valve having a compressed state and an expanded state, and a construction that has a non-uniform expansion resistance profile along its axial length. An expansion member is disposed within the prosthetic heart valve in its compressed state and is capable of applying radially outward forces to the heart valve to convert it to its expanded state. The expansion member is configured to apply non-uniform radially outward forces to the heart valve along its axial length. Preferably, the expansion member includes at least one exterior marker for registering the expansion member within the prosthetic heart valve so that a section of the expansion member capable of the greatest expansion can be located within the stiffest portion of the prosthetic heart valve.

In one embodiment, the expansion member is a balloon having at least one section with a larger expanded diameter than another section. For instance, the balloon has a conical or stepped-diameter valve contact portion. Desirably, the balloon is made of a material doped with a contrast agent.

Another aspect of the invention is a prosthetic heart valve implantation system comprising a balloon-expandable prosthetic heart valve having an inflow end and an outflow end, the heart valve including an outer stent and inner flexible leaflets attached to the stent and a non-uniform expansion resistance profile along its axial length. The prosthetic heart valve may be stiffer at its inflow end than at its outflow end, such as having attachment structure between the leaflets and stent concentrated more heavily adjacent the inflow end of the heart valve than the outflow end. A balloon disposed within the prosthetic heart valve has a non-cylindrical expanded profile with a larger diameter section positioned within a stiffer portion of the heart valve and a smaller diameter section positioned within a more flexible portion of the heart valve. For instance, the balloon has a conical or stepped-diameter valve contact portion. Desirably, the balloon is made of a material doped with a contrast agent. Also, the balloon may include at least one exterior marker for registering the balloon within the prosthetic heart valve so that a section of the balloon capable of the greatest expansion can be located within the stiffest portion of the prosthetic heart valve.

A method of implanting a prosthetic heart valve is also disclosed. The method includes providing a balloon-expandable prosthetic heart valve having a compressed state and an expanded state, and a construction that has a non-uniform expansion resistance profile along its axial length. An expansion member is provided within the prosthetic heart valve in its compressed state. The combined prosthetic heart valve and expansion member are delivered to a target annulus, and non-uniform radially outward forces are applied with the expansion member to the heart valve along its axial length to convert the heart valve to its expanded state.

The expansion member may be a balloon having at least one section with a larger expanded diameter than another section. Preferably, the balloon is made of a material doped with a contrast agent, in which case the step of applying non-uniform radially outward forces to the heart valve consists of filling the balloon with saline without any contrast media. The expansion member may include at least one exterior marker, the method further including registering the expansion member within the prosthetic heart valve so that a section of the expansion member capable of the greatest expansion can be located within the stiffest portion of the prosthetic heart valve. Preferably, the expansion member is a balloon, and there are two markers indicting an axial position on the balloon for the prosthetic heart valve. Further, the markers may indicate which orientation the valve should be positioned on the balloon.

A further understanding of the nature and advantages of the present invention are set forth in the following description and claims, particularly when considered in conjunction with the accompanying drawings in which like parts bear like reference numerals.

### Brief Description of the Drawings

Features and advantages of the present invention will become appreciated as the same become better understood with reference to the specification, claims, and appended drawings wherein:
Fig. 1A is a perspective view of an exemplary expandable prosthetic heart valve formed of a generally cylindrical outer stent with leaflets attached therewithin;
Figs. 1B and 1C are expanded and compressed side elevational views, respectively, of the prosthetic heart valve of Fig. 1A;
Fig. 2A is a side elevational due of an exemplary expandable prosthetic heart valve in a compressed state mounted around a cylindrical balloon on a catheter;
Figs. 2B and 2C are two stages in the expansion of the prosthetic heart valve of Fig. 2A illustrating how a cylindrical balloon can detrimentally flare one end thereof; and
Figs. 3A-3D are side elevational views of exemplary prosthetic heart valve expansion balloons in accordance with the present invention.

### Detailed Description of the Preferred Embodiments

The present invention provides an improved system for deploying plastically-expandable prosthetic heart valves so that they assume their intended operating shape. Expandable heart valves have outer frames or stents supporting inner flexible leaflets that provide fluid occluding surfaces. The valves are designed to expand from a compressed state for delivery into an operating shape that ensures good coaptation of the leaflets. That is, the leaflets must come together to prevent backflow or regurgitation of blood, and any misalignment of the deployed stent can compromise the efficacy of the valve. Most expandable prosthetic heart valves have stents that assume substantially the tubular operating shapes, although other final shapes are encompassed by the present invention.

The invention described herein provides a solution that ensures proper deployment of "plastically-expandable" prosthetic heart valves. This term encompasses balloon-expandable prosthetic heart valves, but should not be considered limited to those expanded only with balloons. Although balloons are the accepted method for expansion of such heart valves, other deployment mechanisms such as radially expandable mechanical fingers or other such devices could be used. In this sense, therefore, "plastically-expandable" refers to the material of the frame of the heart valve, which undergoes plastic deformation from one size to a larger size. Examples of plastically-expandable frame materials are stainless steel, Elgiloy (an alloy primarily composed of cobalt, chromium and nickel), titanium alloys, and other specialty metals. For the sake of convention, however, the term "balloon-expandable" prosthetic heart valves will be used primarily herein, but such should be considered to represent "plastically-expandable" heart valves.

The invention accommodates valves of non-constant expansion resistance. That is, the construction details of balloon-expandable prosthetic heart valves are such that one end, typically the inflow end, possesses a greater number of structural components, including stitching. The valves are mounted over an expansion balloon, delivered to the implant site, and the balloon inflated. Because of the axial constructional non-uniformity of most valves, expansion of the balloon will cause more or sooner radial expansion at whichever part of the valve presents the least expansion resistance. Typically, the inflow end presents greater resistance to expansion, resulting in the outflow end being expanded more or faster. The present invention provides a number of differently shaped balloons to accommodate this constructional non-uniformity, so that the valve expands to its designed operational shape. The final shape of the valve stent may be tubular, but it could also be slightly conical or have a non-linear profile. Those of skill in the art will understand that, given the valve properties and desired final shape, an appropriate expansion member (balloon) can be selected for any number of valves.

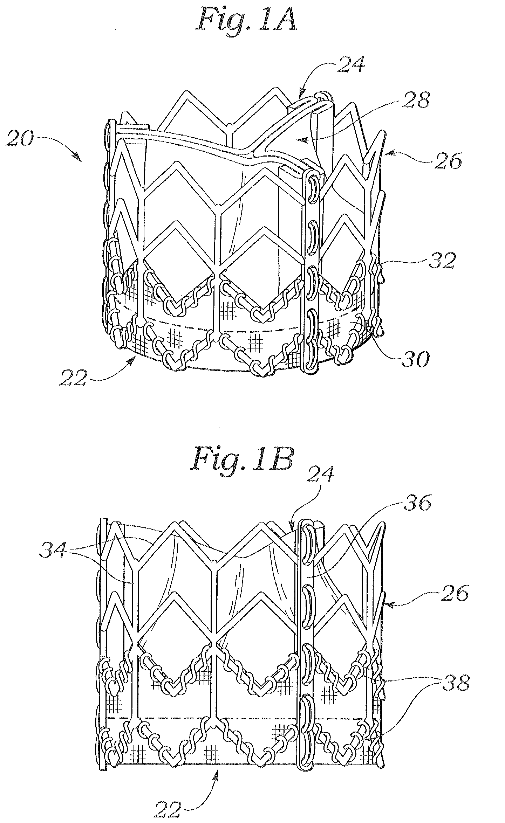

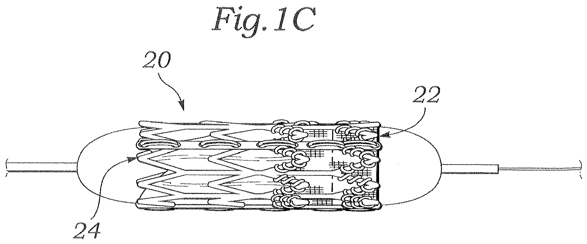

Figs. 1-3 illustrates an exemplary balloon-expandable prosthetic heart valve 20 having an inflow end 22 and an outflow end 24. The valve includes an outer frame or stent 26 supporting a plurality of flexible leaflets 28 within. Fig. 1A shows the valve 20 in its expanded or operational shape, wherein the outer stent 26 generally defines a tube and there are three leaflets 28 attached thereto and extending into the cylindrical space defined within to coapt against one another. In the exemplary valve 20, three separate leaflets 28 are each secured to the stent 26 and to the other two leaflets along their lines of juxtaposition, or commissures. Of course, a whole bioprosthetic valve such as a porcine valve could also be used. In this sense, "leaflets" means separate leaflets or the leaflets within a whole xenograft valve.

In the exemplary valve 20, the flexible material forming the leaflets 28 attaches to the outer stent 28 via a fabric intermediary 30 and a plurality of sutures 32. With reference to Fig. 1B, the stent 26 is formed by a plurality of axially-oriented and circumferentially angled struts 34. At three evenly-spaced points around the stent 26, the axially-oriented struts are replaced by more substantial axial bars 36. The bars 36 include a series of through holes that receive the sutures 32 holding the commissures of the leaflets 28 in place. Additionally, two rows of zig-zag suture lines 38 attached to the two rows of angled struts 34 closest to the inflow end 22. Further details on the exemplary prosthetic heart valves of a similar type can be found in U.S. Patent No. 6,730,118. In addition, the Cribier-Edwards™ Aortic Percutaneous Heart Valve available from Edwards Lifesciences of Irvine, CA is another balloon-expandable prosthetic heart valve of a similar nature, whose construction is also expressly incorporated by reference herein.

As can appreciated by the drawings, the bulk of the attachment structure between the outer stent 26 and inner leaflets 28 is located close to the inflow end 22. Each one of the leaflets 28 desirably connects along an arcuate line between two points at the outflow end 24. This arcuate line passes close to the inflow end 22, and thus the need for more sutures and that end. As a result, the valve 20 has a nonuniform expansion profile. More particularly, the inflow end 22 of the valve 20 exerts substantially greater resistance to expansion on a balloon inflated from within than the outflow end 24. A cylindrical balloon inflated from within the valve 20 with thus expand faster or farther at the outflow end 24 that the inflow end 22, because the outflow and presents the path of least resistance. Fig. 1C shows the valve 20 mounted on a balloon that has been partially inflated.

At this point, it is important to emphasize that the exemplary heart valve 20 is representative of balloon-expandable heart valves that have non-uniform expansion resistance profiles along their axial lengths. In the illustrated embodiment, the valve 20 is stiffer near its inflow end 22 than its outflow end 24. However, other the valves might include an outer frame and an inner valve or leaflet structure which is mounted near the outflow end of the frame, so as to be accordingly stiffer near its outflow end. The term that encompasses these different constructions and other valves is "valves having non-uniform expansion resistance profiles along their axial lengths."

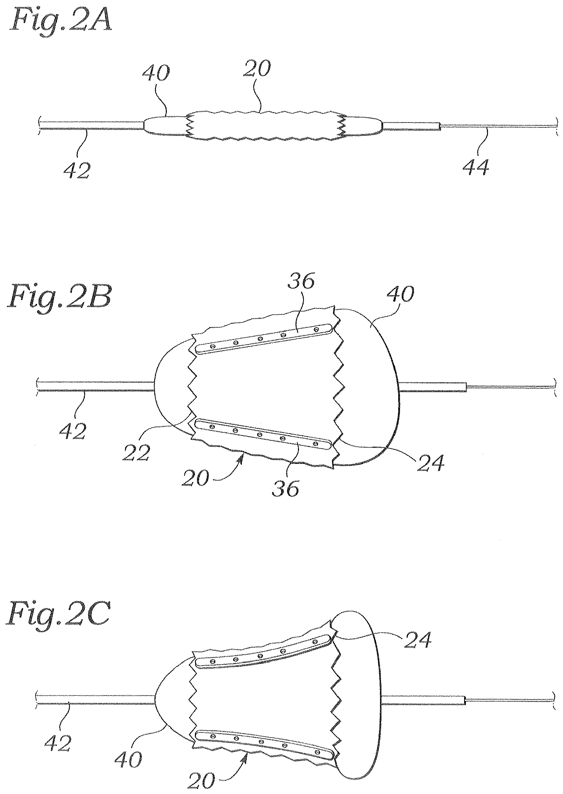

A schematic illustration of this non-uniform valve expansion is seen in Figs. 2A-2C. Fig. 2A shows the valve 20 and its compressed state and crimped over a standard cylindrical balloon 40. The balloon 40 typically mounts on a catheter body 42 that passes over a guidewire 44 to the implant location.

At the implant location, the balloon 40 inflates to deploy the valve 20. Fig. 2B illustrates one possible outcome; the valve expanding into a conical shape with the outflow end 24 expanding faster and farther than the inflow end 22 because of the lesser resistance to expansion at the outflow end. Alternatively, Fig. 2C illustrates a situation in which the outflow end 24 becomes flared or crowned by initial expansion of the balloon 40 beyond the end of the valve. In either of these scenarios, the valve 20 does not expand to its intended cylindrical shape and therefore coaptation of the flexible leaflets within may be compromised. For example, the conical shape of the valve 20 in Fig. 28 may separate the outflow end of the bars 36 and leaflet commissures to such a degree that the leaflets no longer meet in the middle of the valve orifice.

As mentioned above, the present invention provides differently-shaped expansion members or balloons to ensure designed expansion of prosthetic heart valves. As mentioned above, balloons are almost universally used to deploy expandable heart valves. However, is conceivable that a mechanical expansion member such as elongated fingers or hydraulically operated expanding members (i.e., not balloons) could be utilized. Therefore, the term expansion members is intended to encompass balloons and other variants.

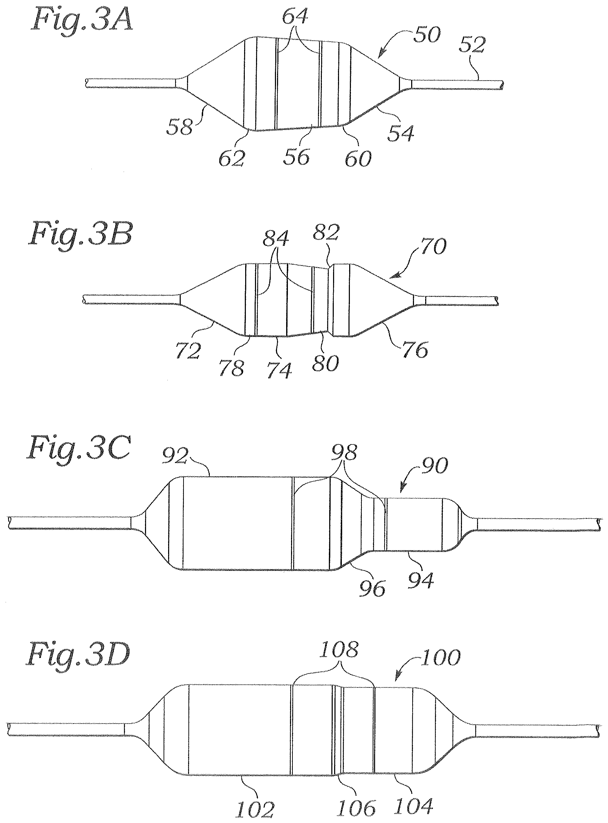

In Fig. 3A a balloon 50 mounts on a catheter 52 and includes a first proximal taper 54, a central valve contact portion 56, and a second, distal taper 58. The valve contact portion 56 has a slight conical taper such that a proximal shoulder 60 has a smaller diameter than a distal shoulder 62. The balloon 50 includes a plurality of marker bands 64 therearound to facilitate registration of a prosthetic heart valve with the balloon.

It is important to note that the terms "proximal and distal" in terms of the balloon tapers is dependent on the direction of heart valve delivery into the annulus, because the heart valve leading end and thus balloon orientation on the catheter will be reversed in a heart valve replacement procedure that begins in a femoral artery as compared to a procedure that enters through the apex of the left ventricle. The balloon 50 of Fig. 3A has a larger distal shoulder 62, which in this embodiment is the end associated with the inflow end of the heart valve 20, indicating that the balloon 50 is oriented for apical delivery as opposed to percutaneous, for which the "proximal" shoulder would correspond to the outflow end of the valve.

For example, the expandable heart valve 20 described above is positioned in its expanded state around the deflated balloon 50. The marker bands 64 are used to position the valve axially on the balloon 54 for proper inflation. Because of the non-uniform expansion profile of the balloon 50, the axial position of the valve 20 is most important to ensure that the portions of the balloon that are capable of applying the largest initial radially outward force are in registry with the stiffer areas of the valve. In particular, the valve 20 is positioned on the balloon 50 such that its inflow end 22 is closer to the first shoulder 62, and its outflow end 24 is closer to the second shoulder 60. Subsequently, the prosthetic valve 20 is crimped around the balloon 50 so as to be ready for delivery into the body and advancement to the target implantation site. When the balloon 50 inflates, the first shoulder 62 initially expands faster and ultimately farther than the second shoulder 60, thus compensating for the increased resistance to expansion of the prosthetic heart valve 20 at its inflow end 22. By careful calculation of the non-uniform resistance of the prosthetic heart valve to expansion, the tapered balloon 50 can be chosen so that the valve expands to its full diameter and proper operational shape (typically a cylinder or a shallow frusto-conical shape).

Fig. 3B shows an alternative heart valve expansion balloon 70 of the present invention having a proximal taper 72, a central valve contact portion 74, and the distal taper 76. Again, the valve contact portion 74 tapers inward from a first shoulder 78 to a second end 80. Instead of then tapering farther down, the distal end of the balloon defines an outwardly formed flange 82 leading to the distal taper 76. Again, marker bands 84 are provided around the circumference of the balloon 70 in the valve contact portion 74 for proper location of the prosthetic valve therearound. The flange 82 further assists in positioning of the valve by providing a clear ridge and tactile marker against which the outflow end 24 of the valve 20 may abut.

Fig. 3C provides a still further balloon 90 of the present invention which includes a proximal section 92 and a smaller distal section 94. A tapered step down 96 connects the proximal section 92 and distal section 94. The marker bands 98 on the balloon 90 generally indicate limits of placement of the prosthetic heart valve therearound. Therefore, the outflow end 24 would be located adjacent the smaller distal section 94, and the inflow end 22 would be located around the larger proximal section 92. In this way, balloon expansion causes more rapid and greater expansion of the inflow end 22 than the outflow end 24. By controlling the relative diameters of the proximal and distal sections 92, 94, the particular prosthetic heart valve can be expanded into its designed cylindrical shape.

Finally, Fig. 3D illustrates a balloon 100 having a proximal section 112 and a distal section 104 separated by a small step 106. The markers 108 indicate where the valve should be placed. The stepped balloon 100 is similar to the balloon 90 of Fig. 3C, but the two sections 102, 104 are closer to each other in diameter.

As will be appreciated by those of skill in the art, the specific shape of the expansion member/balloons described herein will differ depending on the valve construction. Using the exemplary prosthetic heart valve 20, the diameter of the largest part of the balloon that contacts the stiffest portion (e.g., inflow end) of the valve should be greater than the smallest part that contacts the more flexible portion (e.g., outflow end) of the valve. For example, the proximal section 92 in Fig. 3C might be 25 mm in diameter, while the distal section 94 might be 21 mm in diameter. Or, in relative terms, the largest section of the expansion member that contacts the valve is between about 20-30% larger than the smallest section.

The various markers or marker bands disclosed in Figs. 3A-3D may be uniformly circular lines drawn around the balloons to denote placement of the opposite ends of the valve. Alternatively, the markers may be different sizes or configurations to more clearly indicate which orientation the valve should be positioned on the balloon. A more explicit system which includes the words INFLOW and OUTFLOW may also be used for further clarity. Furthermore, the markers may not be axi-symmetric around the balloons, but instead may indicate commissure points or other locations around the balloon with which the valve will register. Indeed, it is also contemplated that the expansion members/balloons may be configured to expand with a non-uniform profile around their circumference. For example, expandable prosthetic heart valves may have non-uniform expansion profiles around their circumference which otherwise would lead to noncircular expansion shapes. Greater outward pressure at the stiffer areas may be required, thus necessitating a particular circumferential registry between valve and balloon as well.

In a typical operational sequence, a prosthetic heart valve having biological tissue thereon is packaged in a separate sterile container from the balloon. In the operating room, the valve and balloon are conjoined for implantation. This procedure requires careful positioning of the valve in its expanded state around the balloon, and crimping of the valve onto the balloon to a predetermined maximum diameter. The marker bands described above therefore greatly facilitate the step of positioning the valve over the balloon to ensure proper expansion. The valve and balloon combination is then inserted into the body and advanced to the target implantation site. The path of delivery may be a relatively long percutaneous route, or may be substantially shorter through a direct-access port or channel in the chest. It is even contemplated that conventional open-heart surgery utilizing cardiopulmonary bypass may benefit from deploying expandable valves by reducing the procedure time.

An alternative use of the present invention is to plastically-expand a prosthetic heart valve that has been partially deployed at the target implantation site. Some self-expanding prosthetic heart valves require further balloon expansion to plastically deform their support frames and ensure proper engagement with the surrounding tissue. The invention therefore encompasses final plastic deformation of initially elastically-expandable frames. In another situation, a purely plastically-expandable heart valve may be partially expanded by a first balloon, and then a second balloon used to completely expand it into its final implant state. In this type of procedure, the marker bands described above are essential to position the non-uniform balloon within the partially deployed valve.

Conventional balloons used to deploy prosthetic heart valves are made of clear nylon. Nylon balloons have a maximum expansion diameter which is important to avoid over-inflation and rupture. In the prior art the inflation fluid consists of a mixture of saline and a contrast media, typically a viscous semi-radiopaque liquid. The inherent viscosity of this fluid increases the inflation/deflation time of the balloon, which is detrimental because the balloon can occlude the aortic annulus for long periods of time.

The present invention provides inflation balloons that are doped with a radiopaque material. The doping is typically performed prior to balloon extrusion to ensure uniform distribution of the doping agent. Consequently, because the balloon itself is radiopaque, saline can be used to inflate it without addition of a viscous contrast media. Because of the lower viscosity of saline, the inflation/deflation time is greatly reduced.

While the invention has been described in its preferred embodiments, it is to be understood that the words which have been used are words of description and not of limitation. Therefore, changes may be made within the appended claims without departing from the true scope of the invention.

## Claims

1. A prosthetic heart valve (20) implantation system, comprising:
a balloon-expandable prosthetic heart valve (20) having a compressed state and an expanded state, and a construction that has a non-uniform expansion resistance profile along its axial length; and
an expansion member (40, 50, 70, 90, 100) disposed within the prosthetic heart valve (20) in its compressed state and capable of applying radially outward forces to the heart valve to convert it to its expanded state,
**characterized by** the expansion member (40, 50, 70, 90, 100) being configured to apply non-uniform radially outward forces to the heart valve (20) along its axial length.

2. The system of claim 1, wherein the expansion member (40, 50, 70, 90, 100) is a balloon (40, 50, 70, 90, 100) having at least one section with a larger expanded diameter than another section.

3. The system of claim 1, wherein the expansion member (40, 50, 70, 90, 100) includes at least one exterior marker (64, 84, 98, 108) for registering the expansion member (40, 50, 70, 90, 100) within the prosthetic heart valve (20) so that a section of the expansion member (40, 50, 70, 90, 100) capable of the greatest expansion can be located within the stiffest portion of the prosthetic heart valve (20).

4. The system of claim 1, wherein the balloon-expandable prosthetic heart valve (20) has an inflow end (22) and an outflow end (24), the heart valve (20) including an outer stent and inner flexible leaflets (28) attached to the stent (26), wherein the expansion member is a balloon (40, 50, 70, 90, 100), and
wherein the balloon (40, 50, 70, 90, 100) has a non-cylindrical expanded profile with a larger diameter section positioned within a stiffer portion of the heart valve (20) and a smaller diameter section positioned within a more flexible portion of the heart valve (20).

5. The system of claim 1, wherein the prosthetic heart valve (20) has a cylindrical expanded state.

6. The system of claim 2 or claim 4, wherein the balloon (40, 50, 70,90, 100) has a conical valve contact portion (56).

7. The system of claim 2 or claim 4, wherein the balloon (40, 50, 70, 90, 100) has a stepped-diameter valve contact portion (96, 106).

8. The system of claim 2 or 4, wherein the balloon (40, 50, 70, 90, 100) is made of a material doped with a contrast agent.

9. The system of claim 4, wherein the balloon (40, 50, 70, 90, 100) includes at least one exterior marker (64, 84, 98, 108) for registering the balloon (40, 50, 70, 90, 100) within the prosthetic heart valve (20) so that a section of the balloon (40, 50, 70, 90, 100) capable of the greatest expansion can be located within the stiffest portion of the prosthetic heart valve (20).

10. The system of claim 4, wherein the prosthetic heart valve (20) is stiffer at its inflow end (22) than at its outflow end (24).

11. The system of claim 10, further including attachment structure between the leaflets (28) and stent (26) concentrated more heavily adjacent the inflow end (22) of the heart valve (20) than the outflow end (24).

## Patentansprüche

1. Implantationssystem für eine prothetische Herzklappe (20), welches umfasst:
eine ballonexpandierbare, prothetische Herzklappe (20) mit einem komprimierten Zustand und einem expandierten Zustand, sowie mit einer Bauweise, die entlang ihrer axialen Länge ein ungleichmäßiges Expansionswiderstandsprofil aufweist; und
ein Expansionselement (40, 50, 70, 90, 100), das im Inneren der prothetischen Herzklappe (20) in deren komprimiertem Zustand angeordnet ist und in der Lage ist, radial auswärts gerichtete Kräfte auf die Herzklappe auszuüben, um sie in ihren expandierten Zustand zu überführen,
**dadurch gekennzeichnet, dass** das Expansionselement (40, 50, 70, 90, 100) dafür konfiguriert ist, auf die Herzklappe (20) entlang deren axialer Länge ungleichmäßige, radial auswärts gerichtete Kräfte auszuüben.

2. System nach Anspruch 1, wobei das Expansionselement (40, 50, 70, 90, 100) ein Ballon (40, 50, 70, 90, 100) ist, der wenigstens ein Teilstück mit einem größeren expandierten Durchmesser als ein weiteres Teilstück aufweist.

3. System nach Anspruch 1, wobei das Expansionselement (40, 50, 70, 90, 100) wenigstens eine äußere Markierung (64, 84, 98, 108) aufweist, um das Expansionselement (40, 50, 70, 90, 100) derart genau in die prothetische Herzklappe (20) einzupassen, dass ein Teilstück des Expansionselements (40, 50, 70, 90, 100), das am weitesten expandierbar ist, im Bereich des steifsten Abschnitts der prothetischen Herzklappe (20) platziert werden kann.

4. System nach Anspruch 1, wobei die ballonexpandierbare prothetische Herzklappe (20) ein Einströmende (22) und ein Ausströmende (24) aufweist, wobei die Herzklappe (20) einen äußeren Stent und an dem Stent (26) angebrachte innenliegende flexible Segel (28) umfasst, wobei das Expansionselement ein Ballon (40, 50, 70, 90, 100) ist, und
wobei der Ballon (40, 50, 70, 90, 100) ein nicht-zylinderförmiges expandiertes Profil mit einem Teilstück mit größerem Durchmesser, das in einem steiferen Abschnitt der Herzklappe (20) positioniert ist, und mit einem Teilstück mit kleinerem Durchmesser, das in einem flexibleren Abschnitt der Herzklappe (20) positioniert ist, aufweist.

5. System nach Anspruch 1, wobei die prothetische Herzklappe (20) einen zylinderförmigen expandierten Zustand aufweist.

6. System nach Anspruch 2 oder 4, wobei der Ballon (40, 50, 70, 90, 100) einen konischen Klappenkontaktabschnitt (56) aufweist.

7. System nach Anspruch 2 oder 4, wobei der Ballon (40, 50, 70, 90, 100) einen Klappenkontaktabschnitt (96, 106) mit einem abgestuften Durchmesser aufweist.

8. System nach Anspruch 2 oder 4, wobei der Ballon (40, 50, 70, 90, 100) aus einem Material gefertigt ist, das mit einem Kontrastmittel dotiert ist.

9. System nach Anspruch 4, wobei der Ballon (40, 50, 70, 90, 100) zumindest eine äußere Markierung (64, 84, 98, 108) aufweist, um den Ballon (40, 50, 70, 90, 100) derart genau in die prothetische Herzklappe (20) einzupassen, dass ein Teilstück des Ballons (40, 50, 70, 90, 100), das am weitesten expandierbar ist, im Bereich des steifsten Abschnitts der prothetischen Herzklappe (20) platziert werden kann.

10. System nach Anspruch 4, wobei die prothetische Herzklappe (20) an ihrem Einströmende (22) steifer als an ihrem Ausströmende (24) ist.

11. System nach Anspruch 10, das ferner eine Befestigungsstruktur zwischen den Segeln (28) und dem Stent (26) aufweist, die nahe dem Einströmende (22) der Herzklappe (20) stärker konzentriert als nahe deren Ausströmende (24).

## Revendications

1. Système d'implantation pour une valve prothétique cardiaque (20), lequel comprend:
une valve prothétique cardiaque (20) expansible par ballonnet, ayant un état comprimé et un état d'expansion ainsi qu'une construction qui présente un profil de résistance à l'expansion non-uniforme le long de sa longueur axiale ; et
un élément d'expansion (40, 50, 70, 90, 100) qui est disposé à l'intérieur de la valve prothétique cardiaque (20) alors que celle-ci se trouve à l'état comprimé et qui est en mesure d'exercer sur la valve cardiaque des forces dirigées radialement vers l'extérieur afin de la faire passer dans son état d'expansion,
**caractérisé par** l'élément d'expansion (40, 50, 70, 90, 100) étant conçu pour exercer sur la valve cardiaque (20) le long de sa longueur axiale des forces non-uniformes dirigées radialement vers l'extérieur.

2. Système selon la revendication 1, dans lequel l'élément d'expansion (40, 50, 70, 90, 100) est un ballonnet (40, 50, 70, 90, 100) qui présente au moins une section avec un diamètre qui, à l'état d'expansion, est plus grand que celui d'une autre section.

3. Système selon la revendication 1, dans lequel l'élément d'expansion (40, 50, 70, 90, 100) comprend au moins un repère extérieur (64, 84, 98, 108) afin de placer ledit élément d'expansion (40, 50, 70, 90, 100) d'une manière telle dans la valve prothétique cardiaque (20) qu'une section de l'élément d'expansion (40, 50, 70, 90, 100) qui est la plus expansible peut être positionnée dans la zone de la partie la plus rigide de la valve prothétique cardiaque (20).

4. Système selon la revendication 1, dans lequel la valve prothétique cardiaque (20) expansible par ballonnet présente une extrémité d'entrée (22) et une extrémité de sortie (24), dans lequel la valve cardiaque (20) comprend un stent extérieur et des feuillets (28) souples fixés, à l'intérieur, sur ledit stent (26), dans lequel l'élément d'expansion est un ballonnet (40, 50, 70, 90, 100) et
dans lequel le ballonnet (40, 50, 70, 90, 100) présente un profil d'expansion de forme non-cylindrique avec une section de diamètre plus grand qui est positionnée dans une partie plus rigide de la valve cardiaque (20) et avec une section de diamètre plus faible qui est positionnée dans une partie plus souple de la valve cardiaque (20).

5. Système selon la revendication 1, dans lequel la valve prothétique (20) présente un état cylindrique d'expansion.

6. Système selon la revendication 2 ou 4, dans lequel le ballonnet (40, 50, 70, 90, 100) présente une partie de contact de valve (56) de forme conique.

7. Système selon la revendication 2 ou 4, dans lequel le ballonnet (40, 50, 70, 90, 100) présente une partie de contact de valve (96, 106) de diamètre graduel.

8. Système selon la revendication 2 ou 4, dans lequel le ballonnet (40, 50, 70, 90, 100) est réalisé en un matériau doté d'une substance de contraste.

9. Système selon la revendication 4, dans lequel le ballonnet (40, 50, 70, 90, 100) comprend au moins un repère extérieur (64, 84, 98, 108) afin de placer ledit ballonnet (40, 50, 70, 90, 100) d'une manière telle dans la valve prothétique cardiaque (20) que la section du ballonnet (40, 50, 70, 90, 100) qui est la plus expansible peut être positionnée dans la zone de la partie la plus rigide de la valve prothétique cardiaque (20).

10. Système selon la revendication 4, dans lequel la valve prothétique cardiaque (20) est plus rigide à son extrémité d'entrée (22) qu'à son extrémité de sortie (24).

11. Système selon la revendication 10, lequel comprend en outre une structure de fixation entre les feuillets (28) et le stent (26), laquelle est plus concentrée au voisinage de l'extrémité d'entrée (22) de la valve cardiaque (20) qu'au voisinage de l'extrémité de sortie (24) de celle-ci.
